(19) Europäisches Patentamt
European Patent Office
Office européen des brevets

(11) Publication number: **0 065 384 A1**

(12) **EUROPEAN PATENT APPLICATION**

(21) Application number: **82302307.2**

(22) Date of filing: **06.05.82**

(51) Int. Cl.³: **C 07 D 499/20**, C 07 D 499/62

(30) Priority: **13.05.81 GB 8114618**

(43) Date of publication of application: **24.11.82**
**Bulletin 82/47**

(84) Designated Contracting States: **BE CH DE FR GB IT LI NL SE**

(71) Applicant: **BEECHAM GROUP PLC, Beecham House Great West Road, Brentford Middlesex (GB)**

(72) Inventor: **Burton, George, 14 Windborough Road, Carshalton Surrey (GB)**
Inventor: **Best, Desmond John, 2A Little Bridges Close, Southwater Sussex (GB)**

(74) Representative: **Hesketh, Alan, Dr. et al, European Patent Attorney Beecham Pharmaceuticals Great Burgh Yew Tree Bottom Road, Epsom, Surrey, KT18 5XQ (GB)**

(54) **Separation process.**

(57) A process for preparing a compound of formula (I) or a pharmaceutically acceptable salt or in vivo ester thereof:

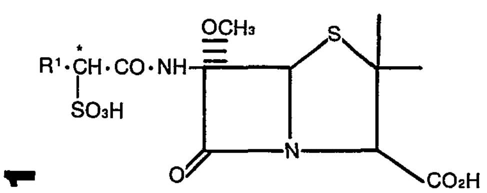

(I)

wherein $R^1$ is $C_{1-6}$ alkyl; an optionally substituted 5-membered heterocyclic ring containing one or two heteroatoms selected from oxygen, sulphur and nitrogen; or phenyl optionally substituted with up to three groups selected from halogen, hydroxy, $C_{1-6}$ alkoxy, nitro, amino, $C_{1-6}$ alkyl, $C_{1-6}$ haloalkyl, $C_{1-6}$ alkylcarbonyloxy, or $C_{1-6}$ alkyl sulphonylamino wherein the carbon atom marked* is in the R-configuration which process comprises inducing or allowing a crystalline precipitate of a compound of formula (II):

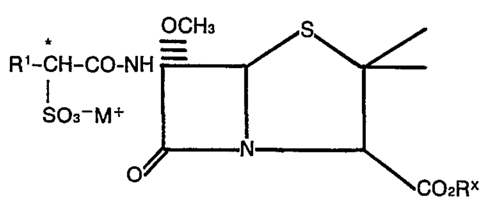

(II)

having R-configuration at carbon atom marked*,
wherein $R^1$ is as defined with respect to formula (I), $R^x$ is a carboxy blocking group and $M^+$ is an alkali metal ion or an optionally substituted ammonium ion; to separate from a solution of a mixture of compounds of formula (II) having R and S configuration at carbon atom marked*;
isolating the crystalline precipitate; and where necessary, under non-racemising conditions, removing the carboxy blocking group $R^x$ and/or forming a free acid, a pharmaceutically acceptable salt or in vivo hydrolysable ester.

EP 0 065 384 A1

ACTORUM AG

## SEPARATION PROCESS

This invention relates to a process for the separation of the components of binary mixtures of penicillin derivatives. In particular, the invention relates to a process for the separation of diastereoisomers of penicillin mixtures.

European Patent Publication No. 0015690 discloses *inter alia* a class of 6-methoxy acylamino penicillins of formula (A) or a pharmaceutically acceptable salt or *in vivo* hydrolysable ester thereof:

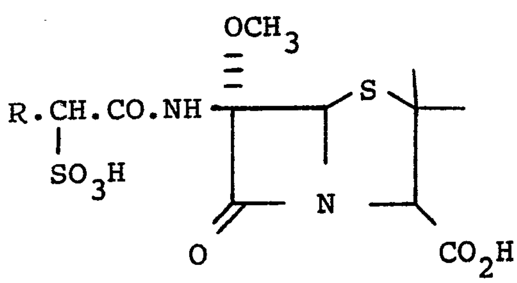

(A)

wherein R is $C_{1-6}$ alkyl; an optionally substituted 5-membered heterocyclic ring containing one or two heteroatoms selected from oxygen, sulphur and nitrogen; phenyl; mono-substituted phenyl where the substituent is halogen, hydroxy, $C_{1-6}$ alkoxy, nitro, amino, $C_{1-6}$ alkyl, $C_{1-6}$ haloalkyl, $C_{1-6}$ alkylcarbonyloxy, or $C_{1-6}$ alkyl sulphonyl-amino (for example - $NHSO_2CH_3$); or di-substituted phenyl where the substituents are selected from hydroxy, halogen, methoxy, acetoxy and amino.

The carbon atom marked * in formula (A) is asymmetric so that the compounds may exist as two optically active diastereoisomers. European Patent Publication No. 0 015 690 further discloses that compounds prepared from the D-side chain exhibit the higher antibacterial activity.

We have now found a facile process for separation of the penicillin derivatives containing the R-side chain from an R, S-mixture. The present process avoids the necessity to perform a resolution of the isomers of the α-sulpho acetic acid derivatives prior to their coupling to the 6,β-amino-6,α-substituted-penicillanic acid nucleus.

Accordingly, the present invention provides a process for preparing a compound of formula (I) or a pharmaceutically acceptable salt or in vivo hydrolysable ester thereof:

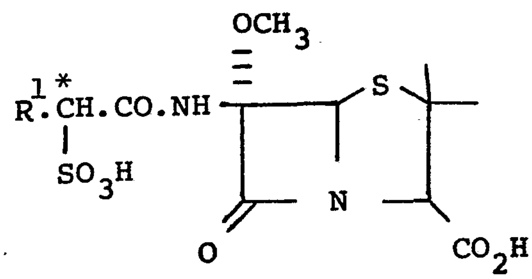

(I)

wherein $R^1$ is $C_{1-6}$ alkyl; an optionally substituted 5-membered heterocyclic ring containing one or two heteroatoms selected from oxygen, sulphur and nitrogen; or phenyl optionally substituted with up to three groups selected from halogen, hydroxy, $C_{1-6}$ alkoxy, nitro, amino, $C_{1-6}$ alkyl, $C_{1-6}$ haloalkyl, $C_{1-6}$ alkylcarbonyloxy, or $C_{1-6}$ alkyl sulphonylamino (for example $-NHSO_2CH_3$) wherein the carbon atom marked * is in the R-configuration which process comprises inducing or allowing a crystalline precipitate of a compound of formula (II):

(II)

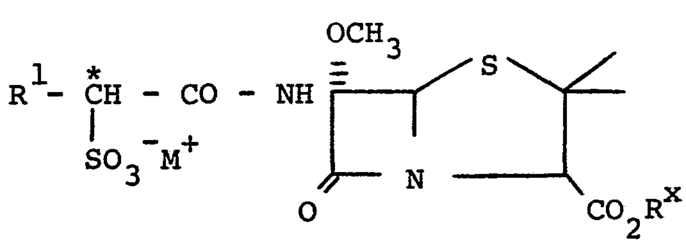

having R-configuration at carbon atom marked *, wherein $R^1$ is as defined with respect to formula (I), $R^x$ is a carboxy blocking group and $M^+$ is an alkali metal ion or an optionally substituted ammonium ion; to separate from a solution of a mixture of compounds of formula (II) having R and S configuration at carbon atom marked *; isolating the crystalline precipitate; and where necessary, under non-racemising conditions, removing the carboxy blocking group $R^x$ and/or forming a free acid, a pharmaceutically acceptable salt or in vivo hydrolysable ester.

The compounds produced by the process of the present invention include the pharmaceutically acceptable esters of compound (I) which hydrolyse readily in the human body to produce the parent acid, for example acyloxyalkyl groups such as acetoxymethyl, pivaloyloxymethyl, α-acetoxyethyl, α-acetoxybenzyl and α-pivaloyloxymethyl groups; alkoxycarbonyloxyalkyl groups, such as ethoxycarbonyloxymethyl and α-ethoxycarbonyloxyethyl; and lactone groups such as phthalidyl or dimethoxyphthalidyl.

Suitable salts of the compound of formula (I) include salts of the 3-carboxylic acid group and also of the sulpho group. Salts include metal salts, eg aluminium, alkali metal salts such as sodium or potassium, alkaline earth metal salts such as calcium or magnesium, and ammonium or substituted ammonium salts, for example those with lower alkylamines such as triethylamine, hydroxy-lower alkylamines such as 2-hydroxyethylamine, bis-(2-hydroxyethyl)-amino or tri-(2-hydroxyethyl)-amine, cycloalkylamines such as bicyclohexylamine, or with procaine, dibenzylamine, N,N-dibenzylethylenediamine, 1-ephenamine, N-ethylpiperidine, N-benzyl-β-phenethylamine, dehydroabietylamine, N,N'-bisdehydroabeitylamine, ethylenediamine, or bases of the pyridine type such as pyridine, collidine or quinoline, or other amines which have been used to form salts with known penicillins.

Suitably $R^1$ is phenyl optionally substituted with up to three groups selected from halogen, hydroxy, $C_{1-6}$ alkoxy, nitro, amino, $C_{1-6}$ alkyl, $C_{1-6}$ haloalkyl, $C_{1-6}$ alkylcarbonyloxy or $C_{1-6}$ alkylsulphonylamino.

More suitably $R^1$ is phenyl; mono-substituted phenyl where the substituent is fluorine, chlorine, bromine, hydroxy, methoxy, nitro, amino, acetoxy or trifluoromethyl; or di-substituted phenyl where the substituents are selected from acetoxy and methoxy.

The compound of formula (II) employed in the process of the present invention may suitably be prepared by the processes disclosed in European Patent Publication No. 0 015 690. Prior to the process of the present invention the compound of formula (II) may be enriched in compound having the R-configuration at the carbon atom marked * by conventional methods such as, for example, chromatography.

Suitable carboxyl-blocking derivatives for the group $-CO_2R^x$ in formula (II) include salts, esters and anhydride derivatives of the carboxylic acid. The derivative is preferably one which may readily be cleaved at a later stage of the reaction. Suitable salts include tertiary amine salts, such as those with tri-lower-alkylamines, N-ethylpiperidine, 2,6-lutidine, pyridine, N-methylpyrrolidine, dimethylpiperazine. A preferred salt is with triethylamine.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofur-2-yl, tetrahydroyran-2-yl, pentachlorophenyl, p-toluenesulphonylethyl, methoxymethyl, a silyl, stannyl or phosphorus-containing group, such as

described above, an oxime radical of formula $-N=CHR^{o}$ where $R^{o}$ is aryl or heterocyclic, or an in vivo hydrolysable ester radical such as defined above.

The carboxyl group may be regenerated under non-racemising conditions from any of the above esters by usual methods appropriate to the particular $R^{x}$ group, for example, mild acid catalysed hydrolysis, or by enzymically-catalysed hydrolysis, or by hydrogenolysis.

Suitable optionally substituted ammonium ions, $M^{+}$, for forming the salts of formula (II) include the ammonium ion and ions obtained by protonation of amines such as, for example, lower alkylamines such as triethylamine, hydroxy-lower alkylamines such as 2-hydroxyethylamine, bis-(2-hydroxyethyl)-amine or tri-(2-hydroxyethyl)-amine, cyclo-alkylamines such as bicyclohexylamine, or with procaine, dibenzylamine, N,N-dibenzylethylenediamine, morpholine, N-methylmorpholine, or bases of the pyridine type such as pyridine, collidine or quinoline, or other amines which have been used to form salts with known penicillins.

Preferably $M^{+}$ is ammonium, triethylammonium or N-methylmorpholinium.

THe separation of the R-isomer according to the present invention is effected through use of an appropriate solvent system. The mixture of diastereoisomers of formula (II) is dissolved in a suitable solvent. After the mixture is totally in solution, crystallisation is induced or allowed, consequently giving precipitation of the resulting crystals. To effect dissolution of the mixture of diastereoisomers, the solvent can be warmed if necessary. If impurities are present so that all of the solids do not dissolve, the undissolved solids can be removed from the liquid by conventional techniques such as settling, filtration or centrifugation.

The appropriate solvent system for use in the present invention is to some extent determined by the choice of the ions, $M^+$, and may be readily found by trial and error. The solvent system may be single or multi component.

Suitable single component solvent systems include $C_{3-10}$ alkanones, such as, for example, acetone and 4-methylpentan-2-one.

Suitable multi component solvent systems are two component systems, such as, for example, a polar organic solvent together with a non-polar diluent.

Suitable polar organic solvents include for example the alkyl esters of alkanoic acids, for example $C_1$ to $C_6$ alkyl $C_1$ to $C_6$ alkanoates, methyl and ethyl $C_6$ to $C_{10}$ alkanoates, $C_6$ to $C_{10}$ alkylacetates, ketones such as $C_3$ to $C_{10}$ alkanones, such as acetone and 4-methylpentan-2-one, water immiscible alcohols such as the $C_5$ to $C_{10}$ alkanols and halogenated hydrocarbons such as chloroform, methylene chloride and ethylene chloride.

Suitable non-polar diluents include the alkanes, toluene, benzene and di-$C_1$ to $C_6$ alkyl ethers. Most convenient are di-$C_{2-4}$ alkyl ethers and those short chain alkanes which are liquid at room temperature (ca 20$^o$ C), for example $C_5$-$C_{10}$ alkanes. Preferred non-polar diluents are diethyl ether and diisopropyl ether.

The temperature at which the dilution and crystallisation is carried out is not critical, but we have found room temperature to be most convenient. It is, however, useful to cool the diluted solution to ensure complete crystallisation.

It is preferred that the solution should be left to stand for some time before the solid is recovered, and crystallisation may be induced by adding a previously obtained seed crystal.

After the crystals have formed, they can be separated from the supernatant liquid by conventional processes. These include settling, filtration and centrifugation.

After separation of the crystals from the supernatant liquid, the solvent is removed from the remaining solute by conventional means, such as evaporation or vacuum evaporation. In some instances, it may be useful to heat the supernatant liquid separated from the crystals to shorten the evaporation time. One of the advantages of the present invention is that the solvent can be recovered from the evaporation step and recycled for further use in the process.

In an advantageous adjunct to the present invention the supernatant liquid, which is enriched in the S-isomer, may be treated as hereinbefore described to yield solute which may be racemised to yield, by the process of the present invention, further compounds of formula (II) wherein the carbon atom marked * is in the R configuration.

Suitable racemisation methods for the compound of formula (II) include base treatment at pH >8, such as, for example, stirring in water at pH 10.

The process of the present invention provides a compound of formula (I) or a pharmaceutically acceptable salt or _in vivo_ hydrolysable ester thereof.

The process further provides crystalline compound of formula (II).

The compounds produced by the present invention are the substantially pure R-isomers. The term 'substantically pure' refers to compound containing at least 90% of the R-isomeric form. More suitably the compound contains at least 95% of the R-isomeric form. Preferably the compound contains at least 99% of the R-isomeric form.

The following Examples illustrate the process of the present invention.

EXAMPLE 1

Disodium 6,β-[R-2-(4-aminophenyl)-2-sulphoacetamido]-6,α-methoxy-penicillanate

(a) Benzyl 6,α-methoxy-6,β-[R-2-(4-nitrophenyl)-2-sulphoacetamido]penicillanate triethyl ammonium salt.

(i) Benzyl 6,α-methoxy-6,β-[R,S-2-(4-nitrophenyl)-2-sulphoacetamido]penicillanate triethylammonium salt (8.24 g) was dissolved in acetone (20 ml) and diluted with diethyl ether ( 20 ml) to give a clear solution. This was seeded with a crystal of benzyl-6,α-methoxy-6,β-[R-2-(4-nitrophenyl)-2-sulphoacetamido]penicillanate triethylammonium salt and left overnight at R.T. The crystals were collected, washed with 1:1 acetone: diethyl ether and dried, yield 4.06 g), $[\alpha]_D^{20}$ 1% MeOH + 226.3°,

ν max (KBr) 1775, 1750, 1690, 1520, 1348 and 1038 $cm^{-1}$; δ $[(CD_3)_2SO]$ 1.18 (9H,t,7Hz,$H\overset{+}{N}(CH_2\underline{CH_3})_3$), 1.23, 1.37 (6H, 2 x s, 2 x $2CH_3$), 3.10 (6H,q,J=7Hz, $H\overset{+}{N}(\underline{CH_2}CH_3)_3$), 3.48 (3H,s,$OCH_3$), 4.50 (1H,s,3H), 5.10 (1H,s,<u>CH</u>CONH), 5.20 (2H,s,$O\underline{CH_2}Ph$), 5.36 (1H,s,5H), 7.40 (5H,s,Ph), 7.78 and 8.15 (4H, ABq,J=8.5Hz, $C_6H_4$), 9.57 (1H,s,CONH).

Found: C 53.00; H 5.92; N 8.16; S 9.27%. $C_{30}H_{40}N_4O_{10}S_2$ requires: C 52.97; H 5.92; N 8.23; S 9.42%.

Hplc analysis ($C_{18}$ 'μ bondapak' reverse phase column eluted with 32.5% acetonitrile in 0.1M sodium acetate pH 5.0) showed no detectable S diastereoisomer.

Mother liquors evaporated to give material enriched in S diastereoisomer, 3.73 g.

(ii) Benzyl 6,α-methoxy-6,β-[2-(4-nitrophenyl)-2-sulphoacetamido]penicillanate triethylammonium salt enriched in S diastereoisomer recovered from mother liquors (4.72 g) was dissolved in water (50 ml), adjusted to pH 10 with triethylamine and stirred at R.T. for 40 minutes. The solutions was adjusted to pH 3 with 5N hydrochloric acid and extracted with chloroform (4 x 50 ml). The extracts were evaporated <u>in vacuo</u> to a foam (4.41 g) which was dissolved in 4-methylpentan-2-one (25 ml), seeded with a crystal of benzyl 6,α-methoxy-6,β-(R-2-(4-nitrophenyl)-2-sulphoacetamido]penicillanate triethylammonium salt. After one hour the crystals were collected, washed with 4-methylpentan-2-one then with diethyl ether and dried, yield 1.53 g, identical with the material obtained in (a) (i).

(b) Disodium 6,β-[R-2-(4-aminophenyl)-2-sulphoacetamido]-6,α-methoxy-penicillantate

Benzyl 6,α-methoxy-6,β-[R-2-(4-nitrophenyl)-2-sulphoacetamido]-penicillanate triethylammonium salt (2.88 g) in aqueous methanol (50 ml) was hydrogenated at atmospheric pressure in the presence of 10% palladium on carbon for 1 hour. The catalyst was filtered off and the filtrate passed through a column of 'Amberlite IR 120 (Na)' and the eluent freeze-dried to give the title compound, 2.09 g.

ν max (KBr) 1765, 1675, 1610, 1515, 1345, 1210 and 1040 $cm^{-1}$;
δ ($D_2O$) 1.28, 1.43 (6H,2 x s, 2 x $2CH_3$),
3.51 (3H,s,$OCH_3$), 4.26 (1H,s,3H), 5.08 (1H,s,<u>CH</u>CONH),
5.56 (1H,s,5H), 6.97 and 7.50 (4H, ABq,J=8.5Hz),$C_6H_4$).

Hplc analysis ($C_{18}$ 'μ bondapak' reverse phase column eluted with 2% methanol in 0.1M sodium acetate, pH 5) showed <1% S diastereoisomer.

EXAMPLE 2

Benzyl 6,α-methylthio-6,β-(R-2-phenyl-2-sulphoacetamido) penicillanate N-methylmorpholinium salt.

Benzyl 6,α-methylthio-6,β-(R-S-2-phenyl-2-sulphoacetamido) penicillanate N-methylmorpholinium salt (1.25 g) was dissolved in acetone (20 ml) and set aside to crystallise. The crystals were collected, washed with acetone then with diethyl ether and dried *in vacuo*, yield 0.35 g, $[\alpha]_D^{20}$ 1% MeOH + 124.7$^o$,

ν (KBr) 1775, 1750, 1680, 1520, 1455, 1168 and 1032 $cm^{-1}$.

Found: C 53.43; H 5.68; N 6.35; S 14.66%, $C_{29}H_{37}N_3O_8S_3$ requires: C 53.44; H 5.72; N 6.45; S 14.76%.

Hplc analysis ($C_{18}$ 'μ bondapak' reverse phase column eluted with 35% acetonitrile in 0.1M sodium acetate, pH 5.0) showed no detectable S diastereoismer.

EXAMPLE 3

Benzyl 6,α-methoxy-6,β-(R-2-phenyl-2-sulphoacetamido) penicillanate N-methylmorpholinium salt.

Benzyl 6,α-methoxy-6,β-(2-phenyl-2-sulphoacetamido) penicillanate N-methylmorpholinium salt (3.76 g) dissolved in acetone (20 ml) and left to crystallise. The crystals were collected, washed with acetone then diethyl ether and dried *in vacuo*, yield 2.01 g $[\alpha]_D^{20}$ 1% MeOH + 220.2°

ν max (KBr) 1775, 1742, 1683, 1455, 1205 and 1035 $cm^{-1}$;
δ $[(CD_3)_2CO]$ 1.30, 1.38 (6H, 2 x s,2 x $2CH_3$),
2.65 (3H,s,morpholine $NCH_3$), 2.8 - 3.1 (4H,m,2 x morpholine $NCH_2$), 3.60 (3H,s,$OCH_3$), 3.7 - 4.0 (4H,m,2 x morpholine $OCH_2$), 4.50 (1H,s,3H), 5.20 (1H,s,CHCONH), 5.28 (2H,s,$OCH_2$Ph), 5.53 (1H,s,5H), 7.3 - 8.0 (10H,m,2 x Ph), 9.40 (1H,s,CONH).

Found: C 54.55; H 5.81; N 6.48; S 9.94%. $C_{29}H_{37}N_3O_9S_2$
requires: C 54.79; H 5.87; N 6.61; S 10.09%.

Hplc analysis ($C_{18}$ 'μ bondapak' reverse phase column eluted with 35% acetonitrile in 0.1M sodium acetate, pH 5) showed no detectable S diastereoisomer.

EXAMPLE 4

Benzyl 6,β-[R-2-(4-bromophenyl)-2-sulphoacetamido]-6,α-methoxypenicillanate triethylammonium salt

Benzyl 6,β-[R,S-2-(4-bromophenyl)-2-sulphoacetamido]-6,α-methoxypenicillanate triethylammonium salt (3 g) was dissolved in acetone (20 ml) and set aside to crystallise. The crystals were collected, washed with acetone then with diethyl ether and dried *in vacuo*, yield 0.7 g, m.p. 185-7$^{o}$,

$\nu_{max.}$ (KBr) 1775, 1735, 1690, 1485, 1235 and 1038 $cm^{-1}$, $\delta[(CO_3)_2SO]$ 1.15 (9H, t, J=7Hz, 3 x $CH_2\underline{CH_3}$), 1.23, 1.35 (6H, 2 x s, 2 x $2CH_3$), 3.04 (6H, q, J=7Hz, 3 x $\underline{CH_2}CH_3$), 3.42 (3H, s, $OCH_3$), 4.46 (1H, s, 3H), 4.81 (1H, s, CHCON), 5.17 (2H, s, $CH_2O$), 5.32 (1H, s, 5H), 7.37, 7.41 (4H, 2 x s, Ar), 9.49 (1H, s, CONH).

The mother liquors were evaporated to dryness and redissolved in water (5 ml). On standing the solution crystallised to yield 0.3 g of the same isomer. Spectral data were the same as above.

EXAMPLE 5

Benzyl 6,β-[R-2-(4-ethoxycarbonyloxyphenyl)-2-sulphoacetamido]-6,α-methoxypenicillanate

Benzyl 6,β-[R,S-2-(4-ethoxycarbonyloxyphenyl)-2-sulphoacetamido]-6,α-methoxypenicillanate triethylammonium salt, (1.95 g) was taken up in warm 4-methylpentan-2-one (50 ml). After standing in the fridge overnight the crystals were filtered, washed with small quantities of cold 4-methylpentan-2-one and diethyl ether and dried to give the title compound as its triethylammonium salt (0.87 g);

$\nu_{max.}$ (Nugol) 1780, 1760, 1745, 1695 $cm^{-1}$; $\delta[(CD_3)_2CO]$, 1.36 (18H, m, 2 x $2CH_3$, $\underline{CH_3}CH_2N\underline{CH_3}CH_2O.CO$), 3.05 (6H, q, J 8Hz, $CH_3\underline{CH_2}N$), 3.56 (3H, s, $OCH_3$), 4.28 (2H, q, J 7Hz, $CH_3\underline{CH_2}O$), 4.46 (1H, s, 3H), 5.10 (1H, s, CHCO), 5.23 (2H, s, $\underline{CH_2}Ph$), 5.46 (1H, s, 5H), 6.8-7.5 (9H, m, Ar), 9.33 (1H, s, NH).

EXAMPLE 6

Disodium 6,β-[R-2-(4-ethoxycarbonyloxyphenyl)-2-sulphoacetamido]-6,α-methoxypenicillanate

Benzyl 6,β-[R-2-(4-ethoxycarbonyloxyphenyl)-2-sulphoacetamido]-6,α-methoxypenicillanate (0.5 g), triethylammonium salt, in 30% aqueous methanol (30 ml) was hydrogenated with 10% palladium on charcoal (0.5 g) for ten minutes at standard temperature and pressure. The mixture was then filtered and passed down a column of Amberlyst IR 120 ($Na^+$) ion-exchange resin. The eluant was collected and evaporated to give the title compound (0.38 g, 96%).

$\nu_{max.}$ (KBr), 1760, 1680, 1605 $cm^{-1}$; $\delta[(CD_3)_2SO]$, 1.32 (9H, m, 2 x $2CH_3$, $\underline{CH_3}CH_2O$), 3.30 (3H, s, $OCH_3$), 3.90 (1H, s, 3H), 4.24 (2H, q, J 7Hz, $CH_3CH_2$), 4.78 (1H, s, CHCO), 5.28 (1H, s, 5H), 7.25 (4H, q, Ar), 9.30 (1H, s, NH).

Claims

1. A process for preparing a compound of formula (I) or a pharmaceutically acceptable salt or in vivo ester thereof:

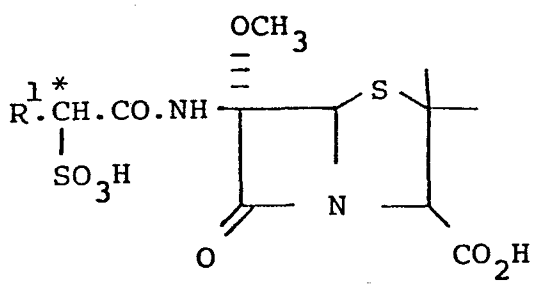

(I)

wherein $R^1$ is $C_{1-6}$ alkyl; an optionally substituted 5-membered heterocyclic ring containing one or two heteroatoms selected from oxygen, sulphur and nitrogen; or phenyl optionally substituted with up to three groups selected from halogen, hydroxy, $C_{1-6}$ alkoxy, nitro, amino, $C_{1-6}$ alkyl, $C_{1-6}$ haloalkyl, $C_{1-6}$ alkylcarbonyloxy, or $C_{1-6}$ alkyl sulphonylamino wherein the carbon atom marked * is in the R-configuration which process comprises inducing or allowing a crystalline precipitate of a compound of formula (II):

(II)

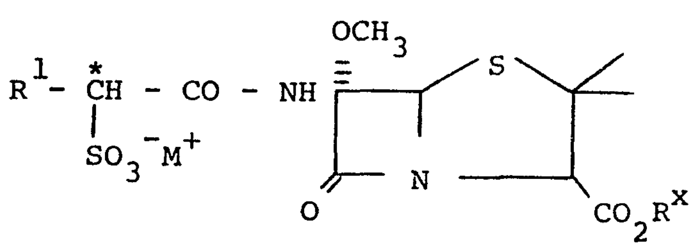

having R-configuration at carbon atom marked *, wherein $R^1$ is as defined with respect to formula (I), $R^x$ is a carboxy blocking group and $M^+$ is an alkali metal ion or an optionally substituted ammonium ion; to separate from a solution of a mixture of compounds of formula (II) having R and S configuration at carbon atom marked *; isolating the crystalline precipitate; and where necessary, under non-racemising conditions, removing the carboxy blocking group $R^x$ and/or forming a free acid, a pharmaceutically acceptable salt or in vivo hydrolysable ester.

2. A process as claimed in claim 1 wherein $R^1$ is phenyl optionally substituted with up to three groups selected from halogen, hydroxy, $C_{1-6}$ alkoxy, nitro, amino, $C_{1-6}$ alkyl, $C_{1-6}$ haloalkyl, $C_{1-6}$ alkylcarbonyloxy, or $C_{1-6}$ alkyl sulphonylamino.

3. A process as claimed in claim 1 or claim 2 wherein $R^1$ is phenyl; mono-substituted phenyl where the substituent is fluorine, chlorine, hydroxy, methoxy, nitro, amino, acetoxy or trifluoromethyl; or di-substituted phenyl where the substituents are selected from acetoxy and methoxy.

4. A process as claimed in any one of claims 1 to 3 wherein $M^+$ is the ammonium ion or an ion obtained by protonation of an amine.

5. A process as claimed in any one of claims 1 to 4 wherein $M^+$ is ammonium, triethylammonium or N-methylmorpholinium.

6. A compound of formula (I) produced by a process as claimed in claim 1.

7. A compound of formula (I) as claimed in claim 6 containing at least 95% of the R-isomeric form.

8. A compound of formula (I) as claimed in claim 6 containing at least 99% of the R-isomeric form.

9. A crystalline compound of formula (II):

(II) 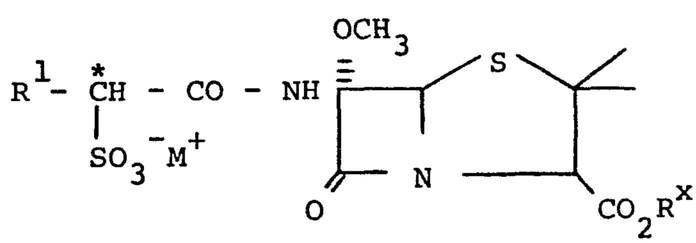

having R-configuration at carbon atom marked *, wherein $R^1$ is as defined with respect to formula (I), $R^x$ is a carboxy blocking group and $M^+$ is an alkali metal ion or an optionally substituted ammonium ion.

10. A pharmaceutical composition comprising a compound as claimed in any one of claims 6 to 8 with a pharmaceutically acceptable carrier or excipient.

European Patent Office

0065384

Application number

# EUROPEAN SEARCH REPORT

EP 82 30 2307

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | JOURNAL OF HETEROCYCLIC CHEMISTRY, vol. 13, no. 3, June 1976, pages 561,562; F.KAJFEZ et al.: "A new synthesis of ampicillin and related investigations". ----- | 1 | C 07 C 499/20<br>C 07 D 499/62 |
| | | | TECHNICAL FIELDS SEARCHED (Int. Cl. 3) |
| | | | C 07 D 499/00<br>A 61 K 31/00 |

The present search report has been drawn up for all claims

| Place of search | Date of completion of the search | Examiner |
|---|---|---|
| THE HAGUE | 23-08-1982 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82